# EUROPEAN PATENT APPLICATION

(11) **EP 4 748 325 A1**
(43) Date of publication of application: **27.05.2026**
(21) Application number: 24844741.9
(22) Date of filing: 19.07.2024
(51) Int. Cl.: A61B 17/34

(54) **DELIVERY DEVICE AND MEDICAL TREATMENT SYSTEM**

(30) Priority: 21.07.2023 CN 202310898704
(71) Applicant: United Innomed (Shanghai) Limited, Shanghai 201315 (CN)
(72) Inventor: XU, Haiyang, Shanghai 201315 (CN); WANG, Li, Shanghai 201315 (CN); LI, Bo, Shanghai 201315 (CN)
(74) Representative: Patentanwälte Olbricht Buchhold Keulertz
(86) International application number: PCT/CN2024/106561
(87) International publication number: WO 2025/021050

(57) **Abstract**

The present disclosure provides a delivery device and a medical system, relates to the technical field of interventional medical instruments, for delivering an implant (10) to a target tissue (20) via a lumen or vessel in a human body. The delivery device comprises a container (100), an outer catheter (200) and a release mechanism (300), wherein the container (100) includes an inner cavity for accommodating the implant (10) and an outlet (110) for discharging the implant; the outer catheter (200) is connected to and communicated with the container (100); the release mechanism (300) includes an inner catheter (310) and a control mechanism (400), and the control mechanism (400) is configured to control an axial relative positional relationship between the inner catheter (310) and the container (100). The present disclosure can achieve the delivery and control of the implant (10) by adjusting the relative position of the inner catheter (310) and the container (100), thereby significantly improving the ease of operation of the delivery device.

## Description

### CROSS REFERENCE TO RELATED APPLICATION

The present disclosure claims the priority of the Chinese patent application for invention with an application number of 202310898704.4, filed on July 21, 2023, and entitled "Delivery Device and Medical System".

### TECHNICAL FIELD

The present disclosure relates to the technical field of interventional medical instruments, particularly to a delivery device and a medical system.

### BACKGROUND

In the field of Class III medical instruments, a delivery sheath is an important medical instrument and mainly used to deliver implants into blood vessels, lumens or other organs of a human body. Among them, the most common implants are stent products, including vascular stents, shunt stents, occluders, artificial valve stents, etc. Other types of implants also include various sensors with anchoring structures and the like. As the structure, configuration, and application of implants become increasingly diverse, current delivery sheaths and their delivery methods are no longer able to meet the needs of some implants. For example, in the prior art, for a shunt stent disposed at an interatrial septum, usually a puncture needle is used to puncture the interatrial septum first to create a perforation, and then the perforation is dilated with a balloon catheter or a dilator to place the shunt stent delivered subsequently. The shunt stent is typically self-expanding in design, which can automatically expand when delivered to the perforation site to engage with the wall of the perforation. For another example, for a sensor with an anchoring structure, the anchoring structure can also have a self-expanding characteristic. For such implants, a delivery sheath is needed that can more quickly and conveniently deliver and release them into place.

### SUMMARY

In order to overcome the above-mentioned defects of the prior art, the present disclosure provides a delivery device and a medical system, for solving one or more problems in the prior art.

In order to achieve the above object, the technical solution adopted by the present disclosure is specifically as follows:
The present disclosure discloses a delivery device for delivering an implant to a target tissue via a lumen or vessel in a human body, wherein the delivery device includes: a container including an accommodating cavity for accommodating the implant and an outlet for discharging the implant; an outer catheter connected to and communicated with the container; an inner catheter extending through the container and the outer catheter and configured to detachably connect with the implant; an end structure provided at a distal end of the inner catheter and configured to selectively block or expose the outlet.

The present disclosure further discloses a medical system comprising an implant and the aforementioned delivery device, wherein the delivery device is used for accommodating and delivering the implant.

The present disclosure further discloses a delivery device for delivering an implant to a target location via a lumen or vessel in a human body, wherein the delivery device includes: a container for accommodating the implant, wherein one end of the container has an outlet for discharging the implant, and the container is provided with a signal transmission window; an outer catheter having a catheter body, a first end and a second end, wherein the outer catheter is connected to and coaxially communicated with the container through the first end; and a release mechanism extending through the outer catheter and the container, wherein the release mechanism is configured to connect with the implant and control an axial relative positional relationship between the implant and the container; wherein the implant includes a wireless sensor, and the release mechanism is configured to keep the implant in the container and align the wireless sensor with the signal transmission window.

The present disclosure further discloses a medical system, comprising: an implant including a wireless sensor; and a delivery device configured to accommodate and deliver the implant, wherein the delivery device is the aforementioned delivery device.

Compared with the prior art, the present disclosure has the following beneficial effects.

When the delivery device according to the embodiments of the present disclosure is in use, the container is connected to and communicated with the outer catheter, and an accommodating cavity for accommodating the implant and an outlet for discharging the implant are formed on the container. In addition, the inner catheter extends through the container and the outer catheter, the implant is detachably connected to the inner catheter, the end structure is disposed at the distal end of the inner catheter, and the end structure is configured to selectively block or expose the outlet. In the present disclosure, the end structure and the implant are both disposed on the inner catheter, and the outlet can be blocked or exposed by using the inner catheter cooperating with the end structure, and the implant is detachably connected to the inner catheter, and when the outlet is exposed, the implant can also be separated from the inner catheter to be released to the target location on the target tissue. The present disclosure can achieve the delivery and control of the implant through an outer catheter and an inner catheter, enabling the implant to be delivered to the target tissue, and the implant can be released without complex operations, thereby significantly improving the ease of operation of the delivery device.

Furthermore, in a specific embodiment, a balloon is used as the end structure which can perform pre-dilation treatment on the target tissue on the delivery path of the implant, thereby facilitating the placement of the implant into the target tissue. Meanwhile, after the implant is delivered and placed into the target tissue, the balloon can also perform post-dilatation treatment directly on the implant on a withdrawal path, such that the implant can be more tightly integrated with the target tissue, thereby achieving rapid, portable, and secure placement of the implant. Throughout the entire process, there is no need to repeatedly move the inner catheter, effectively avoiding potential damage to the human tissue that occur during the movement of the inner catheter.

In another specific embodiment, the inner catheter and a corresponding part of the container are not arranged as a conventional coaxial structure, but with a certain offset, which not only achieves effective loading of an implant with a specific structure (such as an implant formed by combining a stent with a sensor), but also avoids the oversize container from affecting the passing performance of the delivery device.

### BRIEF DESCRIPTION OF DRAWINGS

The following drawings are intended only to schematically illustrate and explain the present disclosure and do not limit the scope of the present disclosure. In the drawings:
FIG. 1 is a schematic structural diagram of a delivery device in an embodiment of the present disclosure;
FIG. 2 is a partial cross-sectional schematic structural diagram of the delivery device in the embodiment shown in FIG. 1 of the present disclosure;
FIG. 3 is a cross-sectional schematic structural diagram of a balloon in an inflated state in an embodiment of the present disclosure;
FIG. 4 is a cross-sectional schematic structural diagram of the balloon in a deflated state in an embodiment of the present disclosure;
FIG. 5 is a cross-sectional schematic structural diagram of the implant in a semi-released state in an embodiment of the present disclosure;
FIG. 6 is a cross-sectional schematic structural diagram of the implant in a fully released state in an embodiment of the present disclosure;
FIG. 7 is a cross-sectional schematic structural diagram of an inner catheter being offset in a container in an embodiment of the present disclosure;
FIG. 8 is a cross-sectional schematic structural diagram of a first bending portion and a second bending portion in an embodiment of the present disclosure, in which the implant is omitted;
FIG. 9 is a side-view schematic structural diagram of a balloon in an embodiment of the present disclosure;
FIG. 10 is a side-view schematic structural diagram of a balloon in an embodiment of the present disclosure;
FIG. 11 is a side-view schematic structural diagram of a balloon in an embodiment of the present disclosure;
FIG. 12 is a side-view schematic structural diagram of a balloon in an embodiment of the present disclosure;
FIG. 13 is a cross-sectional schematic structural diagram of the connecting seat in an embodiment of the present disclosure;
FIG. 14 is a side-view schematic structural diagram of the connecting seat in an embodiment of the present disclosure;
FIG. 15 is a side-view schematic structural diagram showing a second included angle α in an embodiment of the present disclosure;
FIG. 16 is a side-view schematic structural diagram showing a third included angle γ in an embodiment of the present disclosure;
FIG. 17 is a perspective schematic structural diagram of the delivery device in an unreleased state in an embodiment of the present disclosure;
FIG. 18 is a perspective schematic structural diagram of the delivery device in a semi-released state in an embodiment of the present disclosure;
FIG. 19 is a perspective schematic structural diagram of the delivery device in a fully released state in an embodiment of the present disclosure;
FIG. 20 is a perspective schematic structural diagram of the delivery device in a post-dilation state in an embodiment of the present disclosure;
FIG. 21 is a perspective schematic structural diagram of the implant in an embodiment of the present disclosure;
FIG. 22 is a schematic structural diagram of the delivery device in another embodiment of the present disclosure;
FIG. 23 is a partially enlarged cross-sectional view of a distal end of the delivery device in FIG. 22;
FIG. 24 is a further enlarged view of the container of the delivery device in FIG. 23;
FIG. 25 is a schematic structural diagram of a first metal wire mesh on the surface of the container in an alternative embodiment;
FIG. 26 is a schematic diagram of distribution of the first metal wire mesh on the surface of the container according to another embodiment of the present disclosure;
FIG. 27 is a schematic structural diagram of the first metal wire mesh in FIG. 26;
FIG. 28 is a schematic diagram of the internal structure of the distal end of the medical system according to an embodiment of the present disclosure;
FIG. 29 is a partial cross-sectional view of the distal end of the medical system in FIG. 28;
FIG. 30 is a schematic diagram showing the compressed state of the implant in the medical system.

### DETAILED DESCRIPTION

For a clearer understanding of the technical features, objects and effects of the present disclosure, specific embodiments of the present disclosure will now be described with reference to the accompanying drawings.

It should be understood that the term "including" or "comprising" and variations thereof used in the present disclosure are open-ended, i.e., "including or comprising but not limited to". The term "according to" means "at least partially according to". It should be understood that although the terms, such as "first" or "second", may be used in the present disclosure to describe various elements, these elements are not defined by these terms, and these terms are only used to distinguish one element from another. The terms "proximal end" and "distal end" refer to relative orientation, relative position, and direction of elements or actions relative to each other from the perspective of a physician. Although the "proximal end" and "distal end" are not limiting, the "proximal end" generally refers to an end of the medical instrument that is close to the physician during normal operation, while the "distal end" generally refers to an end thereof that first enters the patient's body.

### First Embodiment

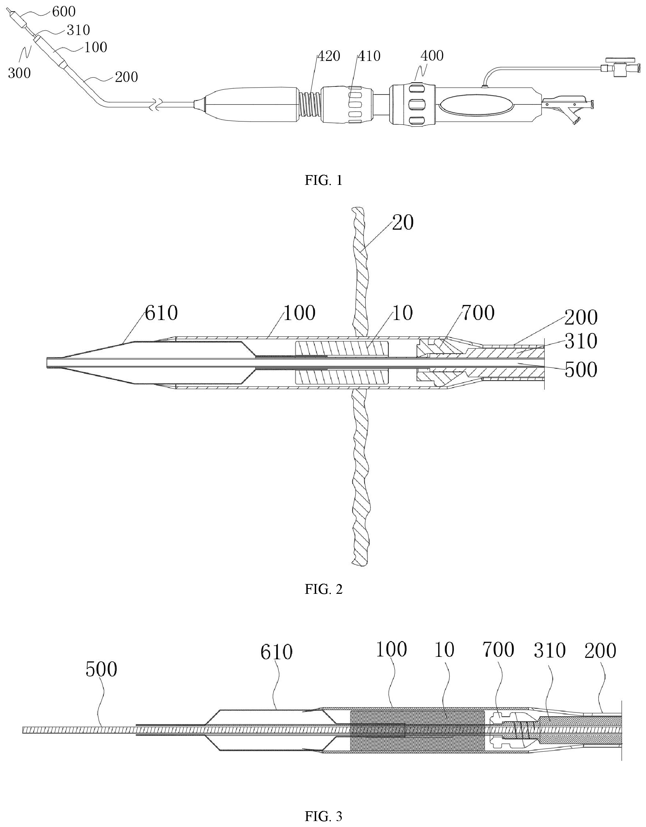

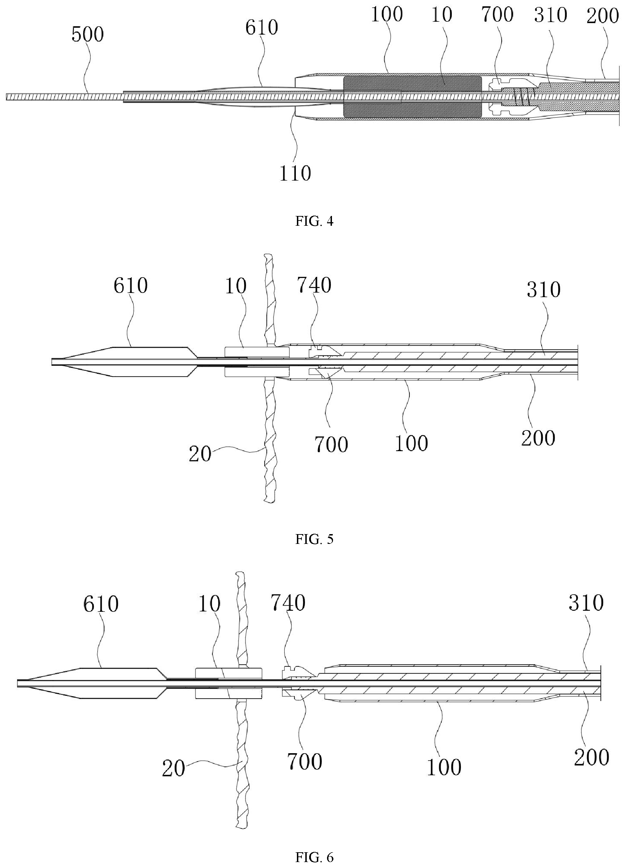

Referring to FIGs. 1, 2 and 21, an embodiment of the present disclosure provides a delivery device for delivering an implant 10 to a target location on a target tissue 20 via a lumen or vessel in a human body. As shown in FIGs. 1 and 2, the delivery device comprises a container 100, an outer catheter 200, an inner catheter 310 and an end structure 600, wherein the container 100 includes an accommodating cavity for accommodating an implant 10 and an outlet 110 for discharging the implant 10 (see FIG. 4); the outer catheter 200 is connected to and communicated with the container 100, and preferably, the outer catheter 200 and the container 100 are integrally formed; the inner catheter 310 extends through the container 100 and the outer catheter 200, and is used to be detachably connected to the implant 10; the end structure 600 is disposed at a distal end of the inner catheter 310, and is used to selectively block or expose the outlet 110 of the container 100.

In the present embodiment, after the implant 10 is loaded onto the inner catheter 310, both the end structure 600 and the implant 10 are disposed on the inner catheter 310, and the implant 10 is located within the container 100. By changing the relative position of the end structure 600 and the container 100, the outlet 110 can be selectively blocked or exposed. Preferably, the implant 10 includes a wireless pressure sensor. When the outlet 110 is exposed, blood can enter the container 100 through the outlet 110 and come into contact with the wireless pressure sensor, thereby achieving intraoperative pressure measurement. The implant 10 is detachably connected to the inner catheter 310, and therefore when the container 100 is gradually separated from the implant 10, the implant 10 can also be gradually separated from the inner catheter 310 to be released to the target location on the target tissue 20. Of course, in other embodiments, the implant 10 can also be an independent stent, but does not include a wireless pressure sensor.

The present disclosure can achieve the delivery and control of the implant 10 by controlling the outer catheter 200 and the inner catheter 310, and the implant 10 can be released without complex operations, thereby significantly improving the ease of operation of the delivery device.

In the embodiment of the present disclosure, as shown in FIGs. 1 and 2, the outer catheter 200 is disposed coaxially with the container 100, and the outer catheter 200 and the container 100 can move axially relative to the end structure 600 such that the end structure 600 blocks or exposes the outlet 110.

It can be understood that in other embodiments, the end structure 600 can also be driven by the inner catheter 310 to move in an axial direction of the container 100 to block or expose the outlet 110.

In order to balance the loading requirement of the implant 10 and the passability of the container 100, the container 100 has a larger size than the outer catheter 200. The container 100 is a circular tube structure with its wall enclosing to form an accommodating cavity, so that the implant 10 can be placed within the container 100.

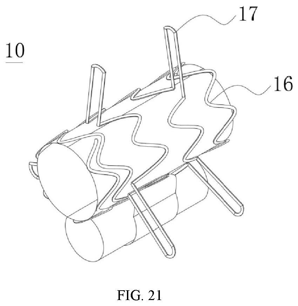

Furthermore, as shown in FIG. 21, the implant 10 includes a stent 16 made of a shape memory material. The stent 16 is adapted to be sleeved over the inner catheter 310 and pressed against the inner catheter 310 under the constraint of the container 100. When the container 100 no longer constrains the stent 16, the stent 16 is capable of self-expanding to a set width and separating from the inner catheter 310.

By utilizing the stent 16 made of a shape memory material, when the stent 16 is detached from the accommodating cavity of the container 100, the stent 16 can separate from the inner catheter 310 and self-expand to a set width, and at this time, the stent 16 can be fixed or preliminarily fixed to the target tissue 20. In the present embodiment, the target tissue 20 is an interatrial septum within the heart, and the stent 16 is a shunt placed in the interatrial septum to guide blood flow from the left atrium to the right atrium. In other embodiments, the stent 16 can also serve as an anchoring structure of other instruments, for anchoring other instruments (such as sensors) to the target tissue 20.

It can be understood that when the distance between the container 100 and the end structure 600 is smaller than a preset value, the distance between the end structure 600 and the implant 10 remains unchanged. The preset value depends on the way of connection between the implant 10 and the inner catheter 310. Specifically, the preset value may be greater than or equal to the distance H1 between the end structure 600 and a component on the inner catheter 310 that is connected to the implant 10, or the preset value may be equal to the distance H2 between the end structure 600 and a proximal end face of the implant 10. When H1 is greater than H2, the preset value is greater than or equal to H1; and when H1 is smaller than or equal to H2, the preset value is equal to H2. It can be understood that only when the container 100 no longer constrains the implant 10 and the implant 10 is separated from the connecting component on the inner catheter 310, the implant 10 is completely released into the target tissue 20. Designers can adjust the specific numerical value of the preset value according to usage requirements (such as the length of the implant 10), there is no specific limitation here. Preferably, the preset value is not greater than the length of the container 100.

More preferably, when at least a portion of the implant 10 is within the container 100, the distance between the container 100 and the end structure 600 can be regarded as being smaller than the preset value. That is, when at least a portion of the implant 10 is within the container 100, and the implant 10 and the inner catheter 310 are in a connected state, the end structure 600 and the implant 10 move synchronously relative to the container 100 and the outer catheter 200, and the distance between the end structure 600 and the implant 10 remains unchanged.

When the distance between the container 100 and the end structure 600 is greater than or equal to the preset value, the implant 10 separates from the inner catheter 310 and self-expands to the set width. As can be seen from the above, when this distance is greater than or equal to the preset value, the implant 10 is removed from the container 100, and at this time, the implant 10 can be placed on the target tissue 20 to support the target tissue 20.

In the embodiment of the present disclosure, the end structure 600 is a plug made of a polymer material. After the implant 10 is released into the target tissue 20, the outer catheter 200 and the inner catheter 310 can be synchronously moved, such that the end structure 600 is withdrawn from the interior of the implant 10 and blocks the outlet 110 of the container 100 again. Then, by moving the outer catheter 200 and the inner catheter 310, the delivery device can be completely withdrawn from the human body.

### Second Embodiment

In the first embodiment, since the end structure 600 is disposed to be unadjustable, after the stent 16 self-expands, if the inner diameter of the stent 16 is smaller than or equal to the outer diameter of the end structure 600, the end structure 600 cannot be withdrawn through the interior of the stent 16, so that the delivery device of such structure can only be suitable for the stent 16 of a specific size, however, the size of the stent 16 is designed according to the needs of holes on the interatrial septum, and the stent 16 will inevitably have different sizes for different groups of people, for example, the size of the stent 16 is relatively small for younger children or adults with small body size. On the other hand, in order to make a stoma on the interatrial septum for placement of the implant 10, in the prior art, a balloon catheter is used to dilate a small hole after puncture, then the balloon catheter is withdrawn, and then the implant 10 is released to the stoma by utilizing the delivery device. The puncture needle, the balloon catheter and the shunt stent 16 all need to be individually delivered into the human body for operation, which not only prolongs the surgical operation time and increases the difficulty of operation, but also brings more operational risks to the surgical operation itself.

In order to overcome the above problems, an improvement has been made to the delivery device in the first embodiment of the present disclosure, and the same contents are not repeated herein, and the differences will be described in detail as follows.

As shown in the embodiments of FIGs. 3 and 4, the end structure 600 includes a balloon 610 which has a first operating state of being in an inflated configuration and a second operating state of being in a deflated configuration. When in the first operating state, the balloon 610 can be used to move relative to the container 100 along the axial direction of the container 100 to block or expose the outlet 110. When the distal end of the delivery device enters the human body and approaches the target tissue 20, the balloon 610 can be used to dilate the puncture hole to form an ideal perforation. After the perforation is formed, there is no need to withdraw the balloon 610, and the delivery device can be continued to be advanced so that the position of the implant 10 corresponds to the perforation, and then the implant 10 can be directly released. In the present embodiment, the location of the perforation is the target location on the target tissue (interatrial septum).

Specifically, in the embodiments shown in FIGs. 5 and 6, the implant 10 can be freed from the constraints of the container 100 by moving the outer catheter 200 and container 100 towards the proximal end. Once the implant 10 is freed from the constraints of the container 100, it can be separated from the inner catheter 310. During the process of separating the implant 10 from the inner catheter 310, the balloon 610 remains stationary and is always closer to the distal end of the implant 10 than the proximal end of the implant 10, while maintaining an unchanged relative axial position with respect to the implant 10.

After the implant 10 is completely separated from the inner catheter 310, regardless of whether the size of the implant 10 after expansion is larger than the size of the balloon 610 in its inflated state, the balloon 610 can transition to the deflated state, so that the balloon 610 can not only be withdrawn through the implant 10 (specifically, it may be the stent 16), but also perform post-dilatation directly on the stent 16 on the withdrawl path, so that the stent 16 can achieve close apposition to the sidewall of the perforation on the target tissue 20.

In other feasible embodiments, when the balloon 610 is in a deflated state during withdrawl, the balloon 610 may not perform post-dilation on the stent 16. It can be understood that physicians can choose whether to use the balloon 610 to expand the stent 16 according to the requirements of the surgical procedure.

In the present disclosure, the balloon 610 and the implant 10 are both disposed on the inner catheter 310, and the inner catheter 310 is utilized to control the state of the balloon 610, so that the balloon 610 can achieve both pre-dilation of the target tissue 20 and post-dilation of the stent 16, and the balloon 610 can freely form an inflated state or a deflated state, such that there is no need to repeatedly deliver and withdraw the balloon catheter, and the delivery device can be applicable to various sizes of the stent 16, thereby greatly simplifying the surgical steps, reducing the pain suffered by patients, and lowering the learning curve for physicians.

In the present embodiment, in order to better exert the post-dilation function of the balloon 610 on the stent 16, the length of the balloon 610 is greater than or equal to the length of the stent 16, so that when the balloon 610 is withdrawn, the balloon 610 can enter the interior of the stent 16 and fully expand the stent 16 in the axial direction of the stent 16.

Furthermore, the nominal width dimension of the balloon 610 after inflation is greater than or equal to the set width of the stent 16, so that after the balloon 610 enters the interior of the stent 16, the balloon 610 can fully expand the stent 16 in a radial direciton of the stent 16, thereby facilitating the stent 16 being installed in place in the target tissue 20. Specifically, since the stent 16 is compressed on the inner catheter 310 during delivery, after the stent 16 is released from the inner catheter 310, the width of the stent 16 after self-expanding (i.e., radial dimension) may be smaller than the set width, or since the size of the perforation does not reach the set width, the width of the stent 16 after release is smaller than the set width. By performing post-dilation on the stent 16 with the balloon 610, the stent 16 can be further expanded to the set width, thereby improving the apposition and secure connection between the stent 16 and the target tissue 20.

In the present embodiment, the delivery device further includes a radiopaque marker disposed on the end structure 600. By providing the radiopaque marker on the end structure 600, when the end structure 600 enters the human body, the radiopaque marker can be visible under X-ray, and the physician can timely and accurately obtain the real-time position of the end structure 600.

Specifically, the end structure 600 includes a balloon 610 on which the radiopaque marker is provided. When the balloon 610 passes through the vascular pathway, the physician can track the relative position of the balloon 610 in real time through the radiopaque marker.

When the balloon 610 is withdrawn into the stent 16, the physician can determine whether the balloon 610 is aligned with the stent 16 based on the radiopaque marker. When the balloon 610 is inside the stent 16, the balloon 610 is further inflated and used to perform post-dilation on the stent 16, thereby allowing the stent 16 to achieve closer apposition to the tissue at the target tissue 20.

Of course, the designers can adjust the specific construction of the radiopaque marker according to usage requirements, for example, the radiopaque marker is at least one radiopaque ring or at least one radiopaque portion, etc., disposed on the end structure 600, which is not specifically limited herein. Preferably, both ends of the balloon 610 are each provided with a radiopaque ring.

When the radiopaque marker is a radiopaque ring, the radiopaque ring cannot display the overall contour state of the balloon 610, that is, the inflated state and the deflated state of the balloon 610 cannot be determined by the radiopaque ring. Therefore, in another feasible embodiment of the present disclosure, the balloon 610 is made of a material with a radiopaque function. By using a material with a radiopaque function to make the balloon 610, the inflated state and the deflated state of the balloon 610 can be displayed. The designers can adjust the specific compositions of the material with a radiopaque function according to usage requirements, which is not specifically limited herein.

In another feasible embodiment of the present disclosure, the balloon 610 may not be provided with a radiopaque marker, but instead the balloon 610 may be visualized under X-ray by injecting a radiopaque agent into the balloon 610 to display the inflated state and the deflated state of the balloon 610. The designers can adjust the specific compositions of the radiopaque agent according to usage requirements, which is not specifically limited herein.

Of course, in other feasible embodiments, the designers can adjust the specific setting manner of the radiopaque marker on the end structure 600 according to usage requirements, which is not specifically limited herein.

When only the radiopaque agent is used, the balloon 610 loses its radiopacity upon deflation and withdrawal, making it impossible to determine the effective position of the balloon 610, while the radiopaque ring can still work at this time. Therefore, the preferred approach is to combine the use of the radiopaque ring and the radiopaque agent.

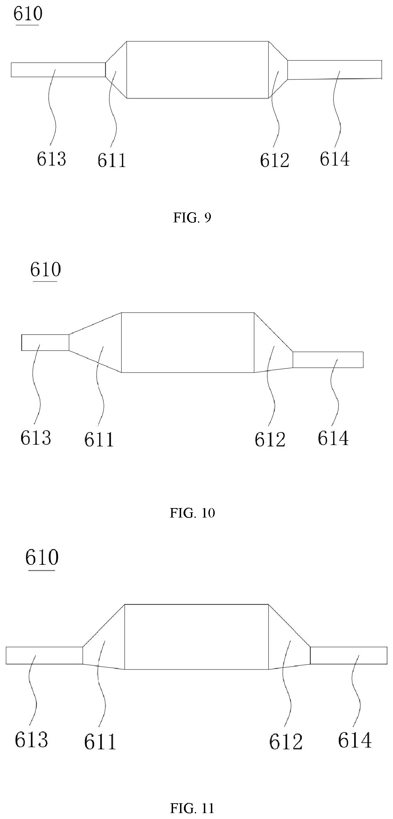

In the embodiments of the present disclosure, as shown in FIGs. 9, 10, 11 and 12, along the direction from the distal end of the inner catheter 310 to the proximal end of the inner catheter 310, the two ends of the end structure 600 respectively form a pre-dilation guide portion 611 and a post-dilation guide portion 612. Along the direction from the post-dilation guide portion 612 to pre-dilation guide portion 611, the cross-section of the pre-dilation guide portion 611 gradually decreases; and/or, along the direction from the pre-dilation guide portion 611 to the post-dilation guide portion 612, the cross-section of the post-dilation guide portion 612 gradually decreases.

By providing the pre-dilation guide portion 611 and the post-dilation guide portion 612 at the two ends of the end structure 600 respectively, and by configuring both the pre-dilation guide portion 611 and the post-dilation guide portion 612 to have a tapered shape, it is beneficial to improve the transition smoothness at the two ends of the end structure 600, such that the end structure 600 can better perform pre-dilation and post-dilation operations, while blocking the outlet 110 of the container 100.

Of course, in other feasible embodiments, the designers can adjust the specific shape and structure of the pre-dilation guide portion 611 and the post-dilation guide portion 612 according to usage requirements, which is not specifically limited herein.

### Third Embodiment

With the advancement of various sensing technologies and processing techniques, in the embodiment as shown in FIG. 21, the implant 10 also exhibits more structural features. For example, when the implant 10 is formed by combining two or more implanting members side by side, the overall radial size thereof will inevitably be larger than that of a single implanting member. As a result, the portion of the delivery sheath that is used to load the implant 10 also had to be correspondingly enlarged, which not only reduces the delivery performance of the delivery sheath, but also increases the risk of causing damage to the human body.

The present embodiment is similar to the first and second embodiments, and the same contents will not be repeated here, and the differences will be described in detail as follows. The implant 10 includes a sensor and a stent 16. After the sensor is combined with the stent 16, the overall volume of the implant 10 is significantly increased compared with a single stent 16, and since the sensor itself is an incompressible rigid member, it is necessary to increase the size of the container 100 of the delivery sheath to provide a sufficient accommodating space for the sensor. In order to avoid enlarging the container 100 as much as possible, the size of the sensor can also be reduced as much as possible through the processing techniques, but this imposes very high demands on technical implementation and processing techniques.

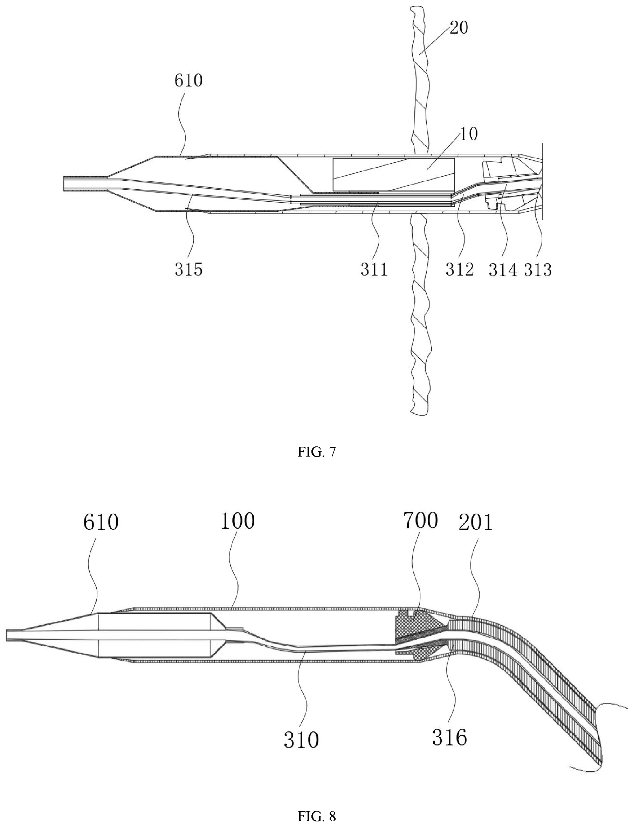

As shown in FIG. 7, the inventors propose a new inner catheter structure, in which the inner catheter 310 is offset in the container 100, thereby causing at least the main body of the implant 10 to be offset in the container 100. Specifically, for a general delivery device, the inner catheter 310 is coaxial with the container 100, and the container 100 is sleeved outside the inner catheter 310 and moves relative to the inner catheter 310, thereby achieving the delivery of the implant 10. However, in this embodiment, a partial segment of the inner catheter 310 that is located in the container 100 (which can be referred to as a loading segment) is no longer coaxial with the container 100, but is offset to one side of the accommodating cavity of the container 10, so that the loading segment of the inner catheter 310 is closer to one sidewall of the container 100, and a larger accommodating space is formed between the inner catheter 310 and the other sidewall of the container 100 for accommodating a rigid sensor. The stent 16 is sleeved over the loading segment, and since the stent 16 is compressible, the stent 16 can still be loaded in the container 100 even if the accommodating space between the inner catheter 310 and one side of the container 100 is small.

By offsetting the loading segment of inner catheter 310 within the accommodating cavity of the container 100, the existing space of the container 100 can be fully utilized, thereby avoiding unnecessarily increasing the overall size of the container 100 and also reducing the requirements for the sensor size.

Furthermore, in the embodiment shown in FIG. 7, along the direction from the distal end to the proximal end of the inner catheter 310, the inner catheter 310 includes a first extension segment 311, a second extension segment 312 and a third extension segment 313. The first extension segment 311 and the third extension segment 313 extend in the same direction, while the second extension segment 312 extends in a different direction from the first extension segment 311 and the second extension segment 312. The first extension segment 311 is a loading segment of the inner catheter 310 for loading the implant 10. The axis of the first extension segment 311 is offset to one side of the axis of the container 100; the implant 10 is disposed on the first extension segment 311, and the stent 16 is sleeved over the first extension segment 311.

Specifically, the first extension segment 311 can be disposed to extend approximately parallel to the axis of the container 100 and offset to one side of the axis of the container 100, and the third extension segment 313 can be disposed to extend substantially along the axis direction of the container 100, or substantially along the axis direction of the outer catheter 200, so that the first extension segment 311 and the third extension segment 313 are offset from each other in the axial direction of the container 100.

By providing the first extension segment 311 and the third extension segment 313 to be offset from each other in the axial direction of the container 100, the first extension segment 311 is offset to one side of the axis of the container 100, thereby being able to form a larger accommodating cavity between the first extension segment 311 and the container 100 for placement of the implant 10. This increases the loading performance of the container 100, allowing for better loading of different types of implants 10 without increasing the volume of the accommodating cavity, significantly improving the applicability of the delivery device.

In the embodiments of the present disclosure, a first port portion 613 and a second port portion 614 are formed at two ends of the end structure 600 along the direction from the distal end of the inner catheter 310 to the proximal end of the inner catheter 310.

Specifically, the first port portion 613 is substantially cylindrical, and a channel is formed inside the first port portion 613 to be sleeved over the inner catheter 310. And the second port portion 614 is substantially cylindrical, and a channel is also formed inside the second port portion 614 to be sleeved over the inner catheter 310.

Through the first port portion 613 and the second port portion 614, it facilitates an increase in the connection length between the end structure 600 and the inner catheter 310 in the axial direction, thereby improving the installation stability of the end structure 600 on the inner catheter 310. The designers can adjust the specific construction of the end structure 600 according to usage requirements, which is not specifically limited herein.

In a feasible embodiment, as shown in FIG. 9, the first port portion 613 and the second port portion 614 are provided coaxially with the axis of the container 100. By providing the first port portion 613 and the second port portion 614 coaxially with the axis of the container 100, the end structure 600 is substantially symmetrically arranged, reducing the difficulty of processing and manufacturing the end structure 600.

In another feasible embodiment, as shown in FIG. 10, the first port portion 613 is provided coaxially with the axis of the container 311, and the second port portion 614 is provided coaxially with the axis of the first extension segment 311.

Since the first port portion 613 is provided coaxially with the axis of the container 311, and the second port portion 614 is provided coaxially with the axis of the first extension segment 311, the first port portion 613 and the second port portion 614 are offset from each other. Therefore, when the inner catheter 310 passes through the end structure 600, the portion of the inner catheter 310 that connects the first port portion 613 and the second port portion 614 exhibits an inclined configuration relative to the container 100.

By providing the second port portion 614 coaxially with the axis of the first extension segment 311, the first extension segment 311 can be smoothly connected with the end structure 600, reducing or eliminating the radial offset amount between the first extension segment 311 and the end structure 600, thereby allowing the guidewire 500 to pass through the first extension segment 311 and the end structure 600 more smoothly.

In a further feasible embodiment, as shown in FIG. 11, the first port portion 613 and the second port portion 614 are provided coaxially with the axis of the first extension segment 311.

By providing the first port portion 613 and the second port portion 614 coaxially with the axis of the first extension segment 311, a straight channel can be provided inside the end structure 600 to connect the first port portion 613 and the second port portion 614, providing better passability.

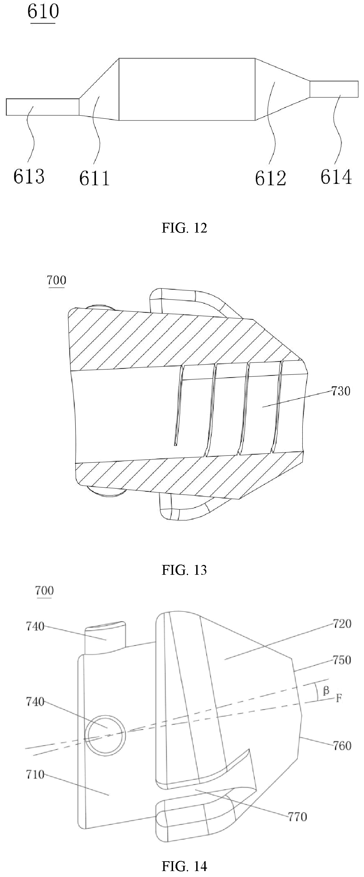

In a further feasible embodiment, as shown in FIG. 12, the first port portion 613 is provided coaxially with the axis of the first extension segment 311, and the second port portion 614 is provided coaxially with the axis of the container 100.

As can be seen from the above, in order to increase the loading performance of the container 100, the axis of the first extension segment 311 is disposed to be offset to one side of the axis of the container 100, resulting in an offset existing between the first extension segment 311 and the axis of the container 100. On one hand, this affects the passability of the guidewire 500 between the first extension segment 311 and the third extension segment 313. On the other hand, since the guidewire 500 needs to pass through the inner catheter 310 and the end structure 600, after the first extension segment 311 is disposed to be offset, it may also easily affect the passability of the guidewire 500 between the first extension segment 311 and the end structure 600.

Therefore, in order to improve the passability between the first extension segment 311 and the end structure 600, it is preferred that the second port portion 614 is provided coaxially with the axis of the first extension segment 311.

By providing the second port portion 614 to be offset on one side of the container 100, the second port portion 614 can reserve more installation space for the implant 10 in the container 100 without affecting the various functions of the balloon 610.

In the embodiments of the present disclosure, as shown in FIGs. 3 and 4, the end structure 600 includes a balloon 610, and the first port portion 613 and the second port portion 614 of the balloon 610 are respectively sleeved over the inner catheter 310; the length of the balloon 610 is greater than or equal to the length of the stent 16; and/or, the nominal width dimension of the balloon 610 after inflation is greater than or equal to the set width of the stent 16, so that when the implant 10 is separated from the inner catheter 310, the balloon 610 can directly expand the stent 16 on the withdrawl path. Regardless of which configuration the balloon 610 takes in FIGs. 9 to 12, the main body of the balloon 610 (i.e., the portion between the first port portion 613 and the second port portion 614) is always disposed coaxially with the cavity of the container 100, thereby allowing the balloon 610 to effectively block the outlet 110 of the container 100.

Specifically, by providing the first port portion 613 and the second port portion 614 at both ends of the balloon 610, the balloon 610 can be connected to the inner catheter 310 in more ways. Different from the existing balloon structure on the catheter, the balloon 610 can be irregularly-shaped balloon 610 (i.e., at least one port portion of the balloon 610 is not coaxial with the main structure), which can selectively block and expose the outlet 110 of the container 100, and meanwhile, by controlling the specific positioning positions of the first port portion 613 and the second port portion 614, there can be better passability between the first extension segment 311 and the balloon 610, facilitating the passage of the guidewire 500.

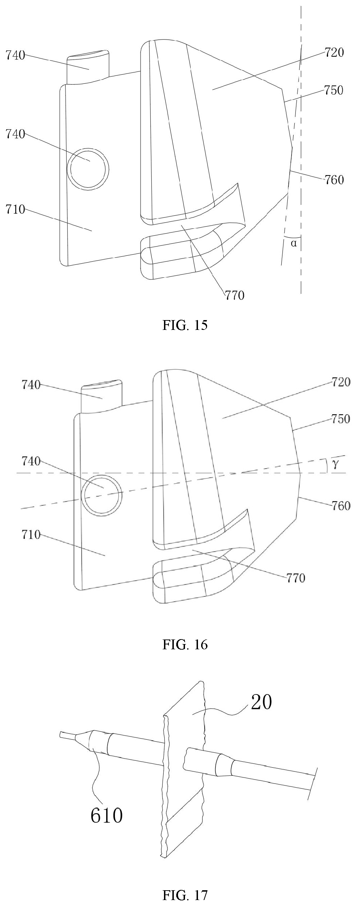

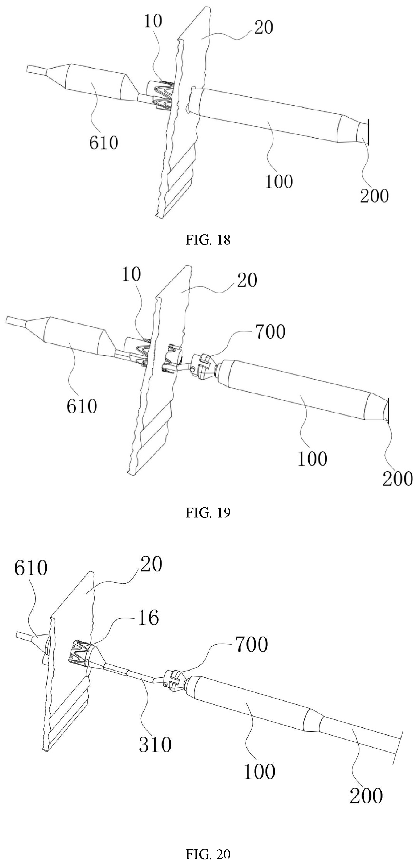

In the embodiments shown in FIGs. 17 to 20, the balloon 610 is provided at the distal end of the inner catheter 310 and enters the human body earlier than the implant 10. After the balloon 610 reaches the target tissue 20, the tissue of the target tissue 20 can be pre-dilated, and for example, a puncture hole on the interatrial septum can be pre-dilated. By controlling the container 100 to withdraw towards the proximal end relative to the inner catheter 310, the implant 10 can be released, allowing the implant 10 to be placed in the interatrial septum.

After the implant 10 is fully released, the inner catheter 310 can also be withdrawn such that the balloon 610 is withdrawn to the location of the stent 16 of the implant 10. When it is determined according to the radiopaque marker that the implant 10 has reached the location of the stent 16, the balloon 610 is further inflated and used to perform post-dilation on the stent 16, thereby achieving closer apposition between the stent 16 and the target tissue 20. It can be understood that in other embodiments, the end structure 600 can also be a common polymer plug.

In the embodiments of the present disclosure, as shown in FIGs. 5, 6, 14, 16 and 21, the delivery device further comprises a first connection structure 740 provided on the inner catheter 310, and the implant 10 comprises an implant body and a second connection structure 17 (see FIG. 21, for simplicity, not shown in FIGs. 5, 6, 14, 16, etc.). When the implant 10 is in the container 100, the implant 10 is connected to the first connection structure 740 through the second connection structure 17 to maintain the axial relative position between the implant 10 and the inner catheter 310. When the implant 10 disengages from the container 100, the second connection structure 17 and the first connection structure 740 can be separated automatically to release the implant 10.

Specifically, the implant 10 includes a stent 16 made of a shape memory material, wherein the stent 16 is provided thereon with a second connection structure 17, and the inner catheter 310 is provided thereon with a first connection structure 740.

In a feasible embodiment, the second connection structure 17 includes a clamping arm provided on the stent 16, and the first connection structure 740 includes a connection block disposed to be protruding. Furthermore, a plurality of clamping arms may be provided, and the connection blocks are correspondingly disposed relative to the clamping arms, thereby improving the stability of the connection between the implant 10 and the inner catheter 310 through multiple groups of clamping arms and connection blocks. Since the clamping arm of the stent 16 is used to clamp on the interatrial septum, the clamping arm is used as the second connection structure 17 which is connected to the first connection structure 740, which can effectively simplify the connection between the implant 10 and the inner catheter 310, and save the internal space of the container 100.

In the present embodiment, the preset value corresponding to the distance between the container 100 and the end structure 600 is equal to the distance between the end structure 600 and the proximal end face of the first connection structure 740 and the width of the clamping arm. After the container 100 is moved to the proximal end face of the first connection structure 740, the container 100 abuts against an end of the clamping arm. Once the container 100 continues to move towards the proximal end and leaves the clamping arm, the stent 16 is completely released.

Of course, in other feasible embodiments, the designers can adjust the specific constructions of the first connection structure 740 and the second connection structure 17 according to usage requirements, which is not specifically limited herein.

When the stent 16 is within the container 100, the container 100 can limit the state of the stent 16, and the stent 16 cannot be unfolded. Then, the stent 16 is connected to the first connection structure 740 through the second connection structure 17 to maintain the axial relative position between the stent 16 and the inner catheter 310.

Generally speaking, after the stent 16 is unfolded, the clamping arm and the main body of the stent 16 exhibit substantially perpendicular configuration. Therefore, when the stent 16 disengages from the container 100, the stent 16 can be unfolded automatically and at the same time drive the clamping arm to return to the predetermined position, causing the second connection structure 17 to be separated from the first connection structure 740, thereby releasing the axial relative position between the stent 16 and the inner catheter 310. Moreover, the stent 16 can self-expand to a set width after being separated from the inner catheter 310, allowing the stent 16 to be placed in the perforation of the target tissue 20.

In the present disclosure, by providing the first connection structure 740 and the second connection structure 17, the structural stability between the implant 10 and the inner catheter 310 is significantly improved, such that the implant 10 can be accurately and quickly released in the target tissue 20, improving the implantation accuracy and portability of the implant 10, effectively simplifying the way of connection between the implant 10 and the inner catheter 310, and saving the internal space of the container 100.

### Fourth Embodiment

The present embodiment is similar to the third embodiment, and the same contents will not be repeated here, and the differences will be described in detail as follows.

Regarding the third embodiment, when the second extension segment 312 is relatively short, the first extension segment 311 and the third extension segment 313 may undergo an abrupt change in the radial direction of the container 100, which may thus easily affect the passability of the guidewire 500 through the inner catheter 310, making it difficult for the guidewire 500 to smoothly pass through the inner catheter 310 and affecting subsequent surgical positioning.

In the present embodiment, as shown in FIGs. 14 to 16, the inner catheter 310 is provided thereon with a connecting member which can be accommodated in the container 100. The connecting member includes a connecting seat 700 on which a first connection structure 740 is provided. An eccentric channel 730 through which the inner catheter 310 passes is formed on the connecting seat 700. The connecting seat 700 is disposed to extend along a first preset direction F, and a first included angle β is formed between the first preset direction F and the axis of the eccentric channel 730, wherein the first included angle β ranges from 0° to 60°, preferably from 15° to 30°. The first preset direction F can be the same as the axis direction of the container 100.

Specifically, the connecting seat 700 can be accommodated within the container 100 and placed between the first extension segment 311 and the third extension segment 313. Moreover, because the first extension segment 311 and the third extension segment 313 are disposed to be offset from each other, in order to avoid the connecting seat 700 from affecting the passability of the inner catheter 310 and further make the transition between the first extension segment 311 and the third extension segment 313 be smoother, an eccentric channel 730 through which the inner catheter 310 passes is formed on the connecting seat 700.

Preferably, in the embodiments shown in FIGs. 8 and 13, the eccentric channel 730 has an inclination tendency from the third extension segment 313 to the first extension segment 311. Moreover, the inner wall of the eccentric channel 730 can be provided with a threaded structure for installation purposes. By providing the eccentric channel 730, the smooth transition length of the inner catheter 310 within the eccentric channel 730 is ensured, thereby improving the passability of the guidewire 500.

Herein, the outer contour of the connecting seat 700 can be coaxially fitted with the container 100, and the first connection structure 740 on the connecting seat 700 can be closely fitted with the inner wall of the container 100, without any gap therebetween, to avoid the second connection structure 17 on the stent 16 from falling off from the gap between the first connection structure 740 and the container 100, thus improving the installation stability between the stent 16 and the connecting seat 700. It can be understood that the container 100 can be made of a polymer material, and the inner wall of the container 100 is smooth. When the first connection structure 740 is in close fit with the inner wall of the container 100, or even in a certain interference fit, the first connection structure 740 and the container 100 can still maintain relative movement.

In other embodiments, the first connection structure 740 on the connecting seat 700 can also be in a clearance fit with the inner wall of the container 100 to improve the passage ability of the connecting seat 700 in the container 100 and avoid interference between the connecting seat 700 and the inner wall of the container 100, which may affect the release of the implant.

The designers can adjust the size of the first included angle β according to usage requirements, for example, the first included angle β can be 10°, 20°, 30°, etc., and there is no specific numerical limit here.

By providing a connecting seat 700 on the inner catheter 310 and providing the first connection structure 740 on the connecting seat 700, thereby avoiding the influence on the structure of the inner catheter 310 and reducing the difficulty of processing and installing the inner catheter 310.

Of course, in other feasible embodiments, the designers can adjust the specific construction of the connecting member according to usage requirements, which is not specifically limited herein.

Furthermore, in the embodiment shown in FIG. 16, the first preset direction F is parallel to the axial direction of the container 100 or the first preset direction F has a third included angle γ with the axial direction of the container 100, wherein the third included angle γ is in the range of 0° to 60°, preferably 15° to 30°.

The designers can adjust the specific size of the third included angle γ according to usage requirements, for example, the third included angle γ can be 5°, 10°, 15°, 20° or 25°, etc., and there is no specific limit here.

In the embodiment of the present disclosure, as shown in FIG. 8, the outer catheter 200 has a first bending portion 201, and the inner catheter 310 has a second bending portion 316. When the end structure 600 blocks the outlet 110, the first bending portion 201 and the second bending portion 316 are correspondingly disposed.

Specifically, the first bending portion 201 is provided to be close to the container 100, and the second bending portion 316 is provided to be close to the connecting member. By providing the first bending portion 201 on the outer catheter 200 and the second bending portion 316 on the inner catheter 310, the first bending portion 201 and the second bending portion 316 cooperate to achieve a predetermined bending angle at the distal end of the delivery device, thereby making it easier for the container 100 to be in place when facing a tissue at a specific position or having a specific structure (such as interatrial septum).

When the outer catheter 200 has a first bending portion 201 and the inner catheter 310 has a second bending portion 316, if the connecting seat 700 is provided at the bending portion, the connecting seat 700 may cause structural interference with the bent part of the container 100, thereby leading to problems, such as damage to the container 100.

In order to solve the above problems, in the embodiments of the present disclosure, as shown in FIGs. 13 and 14, along the direction from the distal end of the inner catheter 310 to the proximal end of the inner catheter 310, the connecting seat 700 includes a first seat body 710 and a second seat body 720 connected with each other. The first seat body 710 is cylindrical, and the first connection structure 740 is disposed on the first seat body 710. The second seat body 720 is frustum-shaped and is connected to the first seat body 710. An eccentric channel 730 extends through the first seat body 710 and the second seat body 720. The second seat body 720 has a first end and a second end, and the radial dimension of the first end is greater than that of the second end.

By forming the eccentric channel 730 on the first seat body 710 and the second seat body 720, compared to a straight channel, the eccentric channel 730 has a longer length, and is provided to be inclined, so as to be able to provide a better smooth transition for the inner catheter 310. This can extend and drive the inner catheter 310 to achieve synchronous unidirectional displacement with the implant 10 in advance, which facilitates reducing the abrupt change in the extension direction of the inner catheter 310 at the connecting seat 700, prevents the guidewire 500 channel within the inner catheter 310 from being lost, and ensures the passability of the guidewire 500 in the inner catheter 310.

In the embodiment shown in FIG. 15, the second end of the second seat body 720 is formed with an orifice end face 750 which is perpendicular to the axis of the eccentric channel 730, and the eccentric channel 730 extends through the orifice end face 750. The second seat body 720 is further provided with an avoidance end face 760, at least a part of which is connected to the orifice end face 750, and the remaining part of the avoidance end face 760 is disposed to be away from the proximal end of the inner catheter 310. A second included angle α is formed between the avoidance end face 760 and the vertical direction, wherein the second included angle α is in the range of 0° to 60°, preferably 15° to 30°. That is, the avoidance end face 760 is disposed to be inclined from the second seat body 720 toward the first seat body 710. The connecting seat 700 can reserve more space inside the container 100 through the avoidance end face 760 to play an avoidance function, which thus facilitate improving the adaptability between the connecting seat 700 and the container 100, reducing or eliminating interference issues therebetween, and preventing damage to the container 100 caused by the connecting seat 700.

The designers can adjust the size of the second included angle α according to usage requirements, for example, the second included angle α can be 10°, 20°, 30°, 40° or 50°, etc., and there is no specific limit here.

Furthermore, in the embodiment shown in FIG. 7, the inner catheter 310 further includes a fourth extension segment 314 which is located between the second extension segment 312 and the third extension segment 313. The fourth extension segment 314 is used to extend through the eccentric channel 730, and an included angle between the fourth extension segment 314 and the axis of the container 100 is smaller than the included angle between the second extension segment 312 and the axis of the container 100.

By controlling the included angle between the fourth extension segment 314 and the axis of the container 100, the inclination amplitude between the fourth extension segment 314 and the second extension segment 312 gradually increases, which facilitates improving the smoothness of the transition between the fourth extension segment 314 and the second extension segment 312, thereby improving the passability of the inner catheter 310.

The designers can adjust the size of the included angle between the fourth extension segment 314 and the axis of container 100, as well as the size of the included angle between the second extension segment 312 and the axis of container 100, according to usage requirements, without specific numerical limitations herein.

Moreover, an end channel is formed on the end structure 600. As shown in the embodiment of FIG. 7, the inner catheter 310 further includes a fifth extension segment 315 which is provided on the other side of the first extension segment 311 relative to the second extension segment 312, and the fifth extension segment 315 extends through the end channel. The inner catheter 310 has an inner cavity that can be used for placement of the guidewire 500.

Specifically, the end channel extends through the first port portion 613, the body of the end structure 600, and the second port portion 614, and the fifth extension segment 315 extends through the end channel.

Since the connecting seat 700 is provided inside the container 100, when the inner catheter 310 and the container 100 move relative to each other in the axial direction, the liquid and/or gas inside the container 100 need to pass through the gap between the connecting seat 700 and the inner wall of the container 100. However, due to limitation of the size of the gap, it is difficult for the liquid and/or gas to efficiently pass through the connecting seat 700, resulting in a damping effect and reducing the motion efficiency between the inner catheter 310 and the container 100.

In order to solve the above problems, in the embodiment of the present disclosure, as shown in FIG. 14, the connecting member is provided thereon with a flow guide structure 770 which communicates the accommodating cavities of the container 100 that are located on both sides of the connecting member.

Specifically, the connecting member includes a connecting seat 700, and the flow guide structure 770 includes a flow guide groove disposed on the connecting seat 700, and the flow guide groove communicates the inner cavities of the container 100 that are located on two sides of the connecting seat 700. The flow-through capacity of liquid and/or gas can be improved by the flow guide groove, thereby reducing or eliminating the damping during the relative motion between the connecting seat 700 and the container 100, and improving the motion efficiency. More preferably, the flow guide groove is provided on the second seat body 720.

In the embodiment of the present disclosure, the end structure 600 includes a balloon 610. The inner catheter is further provided thereon with a flow-through structure which has an injection channel, wherein the injection channel is communicated with the inner cavity of the balloon 610.

Specifically, the flow-through structure includes at least one flow-through port provided on the inner catheter 310, and the flow-through port communicates the injection channel with the inner cavity of the balloon 610, so that the filling fluid can be injected into or discharged from the balloon 610 through the injection channel, thereby facilitating the control of the inflation and deflation state of the balloon 610.

Furthermore, the inner catheter 310 includes a first catheter body and a second catheter body inserted into the first catheter body. The inner cavity of the second catheter body forms a channel of the guidewire 500, and the gap between the first catheter body and the second catheter body forms an injection channel.

Of course, in other feasible embodiments, the designers can adjust the forming method of the injection channel according to usage requirements, without specific restrictions herein.

In the embodiment of the present disclosure, as shown in FIG. 1, the delivery device further comprises a release mechanism 300 which includes an inner catheter 310 and a control mechanism 400. The control mechanism 400 is used to adjust the axial relative position between the inner catheter 310 and the container 100. The control mechanism 400 is configured such that the container 100 can move relative to the inner catheter 310 at the first and second positions in an axial direction. When the container 100 is at the first position, the implant 10 is located inside the container 100. During the process of the container 100 moving from the first position to the second position, the implant 10 is disengaged from the outlet 110 of the container 100.

Specifically, the control mechanism 400 can be a handle. In the present embodiment, the control mechanism 400 includes an actuating member 410 and an auxiliary member 420. The axial position of the auxiliary member 420 is fixed, and actuating member 410 is capable of moving axially on the auxiliary member 420. The actuating member 410 is also connected to the outer catheter 200, so that the actuating member 410 can drive the outer catheter 200 to move axially when the actuating member 410 moves axially. The proximal end of the inner catheter 310 is fixed in the auxiliary member 420 of the control mechanism 400 and maintains an unchanged axial relative position with respect to the auxiliary member 420. In this way, moving the outer catheter 200 forward or backward in the axial direction can cause relative axial movement between the inner catheter 310 and the outer catheter 200. In the present embodiment, the control mechanism 400 can cause a relative position change (relative motion) between the inner catheter 310 and the outer catheter 200 by controlling the movement of the outer catheter 200. In other embodiments, the control mechanism 400 can cause a relative position change (relative motion) between the inner catheter 310 and the outer catheter 200 by controlling the movement of the inner catheter 310.

In the embodiments of the present disclosure, referring to the embodiments shown in FIGs. 17 to 20, specific use steps of a delivery device are further provided. The implant 10 includes a stent and a wireless pressure sensor:

The release mechanism 300 is operated to expose the first extension segment 311 at the distal end of the inner catheter 310, so that the balloon 610 is in a deflated state; the implant 10 is loaded and fixed onto the first extension segment 311, and the first connection structure 740 is connected to the second connection structure 17, then the release mechanism 300 is operated until the implant 10 is completely retracted into the container 100. A pressure filling device is used to empty the handle, the implant 10, and the inner catheter 310 through the Standard Luer connector of the handle. With a pressure filling device with readable pressure values, the balloon 610 is inflated with a 1:1 solution of radiopaque agent and physiological saline to a nominal pressure. Under an image guidance equipment, the delivery system is delivered along the prepared vascular pathway guidewire 500 to the release position of the implant 10 through the guidewire 500. Based on the assessment of the physician and the location of the lesion, if dilation of the lesion pathway or the perforation is required, the lesion pathway or the perforation can be pre-dilated directly by the balloon 610 until the ideal implantation requirement of the implant 10 is achieved. After the positions of the implant 10 and the container 100 are confirmed under the image guidance equipment and by the physician, the balloon 610 is quickly depressurized first by the pressure filling device for real-time pressure detection and reading the pressure value, and then the implant 10 is gradually released until a safe and retrievable point to confirm the accuracy of the implantation position again. If the release position is not ideal, the implant 10 is quickly retrieved at its current location and then withdrawn to a safe position. Subsequently, the balloon 610 is inflated again to the nominal pressure through the pressure filling device, and the above steps are repeated to perform the implantation and release again. After the release position is confirmed to be accurate, the implant 10 is thoroughly released. After the image guidance equipment confirms the accurate release position of the implant 10, under the image guidance equipment, the balloon 610 is first withdrawn to the position corresponding to the implant 10, and then the balloon 610 is inflated by the pressure filling device, achieving post-dilation treatment of the implant 10 and the lesion location. After the post-dilation treatment is completed, the pressure of the balloon 610 is completely released, and then the inner catheter 310 is withdrawn from the human body along the guidewire 500.

### Fifth Embodiment

The present disclosure further provides a medical system comprising an implant 10 and a delivery device as described in any one of the first to fourth embodiments, wherein the delivery device is configured to accommodate and deliver the implant 10.

The specific structure, working principle, and beneficial effects of the delivery device are the same as those of any of the first to fourth embodiments, and will not be repeated here. By using the delivery device in the first embodiment, the medical system can perform pre-dilation treatment on the target tissue 20, and can also perform post-dilation treatment on the implant 10 on the withdrawl path, so that the implant 10 can be better installed in place.

In the embodiments of the present disclosure, the implant 10 includes a stent 16 and a wireless sensor provided side by side with the stent 16. Furthermore, the stent 16 includes a stent main body and a covering membrane, and the stent main body has a second connection structure 17. In a feasible embodiment, the second connection structure 17 includes a clamping arm provided on the stent main body, and the clamping arm can be sleeved over the first connection structure 740 on the connecting seat 700 to achieve that the implant 10 is fixed in the inner catheter 310 and can be released from the inner catheter 310.

In the embodiments of the present disclosure, the medical system further comprises a guidewire 500 for being provided in the inner catheter 310. Specifically, the diameter of the guidewire 500 is smaller than that of the inner catheter 310. The designers can adjust the specific construction of the guidewire 500 according to usage requirements. For example, the guidewire 500 may be a slender metal wire, which is not specifically limited herein. Moreover, the guidewire 500 has a relatively high support strength and a small diameter, so that the guidewire 500 is easier to bend in the human body vessels. The guidewire 500 can play a guiding role, so that when the inner catheter 310 is sleeved over the guidewire 500, the inner catheter 310 can move along the guidewire 500 to the target tissue 20.

Although in the above embodiments, it is taken as an example that the implant is a shunt stent of the interatrial septum and a sensor, those skilled in the art can understand that the delivery device disclosed in the present disclosure can be applied to various implants, including but not limited to a cardiovascular stent.

### Sixth Embodiment

In various scenarios, the physicians need to monitor the physiological parameters of a patient to assess his or her physical condition. If there are abnormalities in the relevant parameters, timely response measures need to be taken. For example, when the patient's left ventricular pressure is excessively high, timely intervention treatment is needed to reduce the left ventricular pressure. In hospitals and other hospital environments, the physician can achieve this goal by placing an interventional catheter with a sensor in the human body, or can implant a wireless sensor in the human body to meet the patient's need for free movement.

But the inventors have found that during the entire implantation process in which the physician implants a wireless sensor or other implants into the human body through the delivery device, it is sometimes necessary to master some physiological parameters in the patient's body. In this case, the conventional approach is to use another set of interventional sensing catheters to place the corresponding sensors at specific positions from other parts of the human body. However, this may not only increase the wound and pain of the patient, but also increase the operation time and consumables costs. On the other hand, due to the differences in implantation process and target position between the interventional sensing catheter placed from other parts and the delivery device, it is inevitable that there will be data deviations, resulting in time delay and low accuracy.

The embodiment of the present disclosure provides a delivery device for delivering an implant to a target location via a lumen or vessel in a human body. The implant can be a wireless sensor used to measure physiological parameters in the lumen or vessel in the human body, or a combination of other implantable element and the wireless sensor, such as a combination of a shunt for atrial shunt surgery and a wireless pressure sensor. The delivery device includes a container, an outer catheter, and a release mechanism. The container is used to accommodate the implant. One end of the container has an outlet for discharging the implant. The container is provided with a signal transmission window. The outer catheter has a catheter body, a first end and a second end. The outer catheter is coaxially communicated with the container through the first end. The release mechanism extends through the outer catheter and the container. The release mechanism is configured to connect with the implant and control the axial relative positional relationship between the implant and the container. The release mechanism is configured such that the implant is kept within the container and the wireless sensor is aligned with the signal transmission window. When performing interventional surgery using the delivery device provided by the embodiment of the present disclosure, the implant can be accommodated in the container and is aligned with the signal transmission window by configuring a release mechanism. In this way, during the implantation process, the implant can also acquire information and transmit the information to the outside of the human body through a signal transmission window, which can obtain immediate and accurate physiological parameters during the intervention process. The alignment or correspondence between the wireless sensor and the signal transmission window as referred to in the present disclosure means that the wireless sensor axially corresponds to the signal transmission window. Furthermore, the wireless sensor can include a signal acquisition unit and a wireless signal transmission unit. Therefore, it is also feasible that only the wireless signal transmission unit in the wireless sensor axially corresponds to the signal transmission window. That is, the signal of the wireless signal transmission unit can be transmitted to an extracorporeal receiving device through the signal transmission window without obstruction, and there is no limitation in the present disclosure.

The delivery device provided in the present disclosure is used to deliver the implant including the wireless sensor to the target locations, so that not only the wireless sensor plays monitoring and diagnosing functions after being implanted, but also by providing a signal transmission window in the container accommodating the implant, during the entire delivery process, the wireless sensor can also play a role in transmitting the sensed information to the outside of the human body through a wireless signal, to achieve real-time and accurate monitoring of human physiological parameters during the intervention process. This eliminates the need to implant other intervention sensing catheters during surgery, which not only reduces the patient's trauma and pain, but also achieves changes in the entire surgical procedure, effectively shortens surgical time, reduces surgical difficulty, and meanwhile reduces surgical costs.

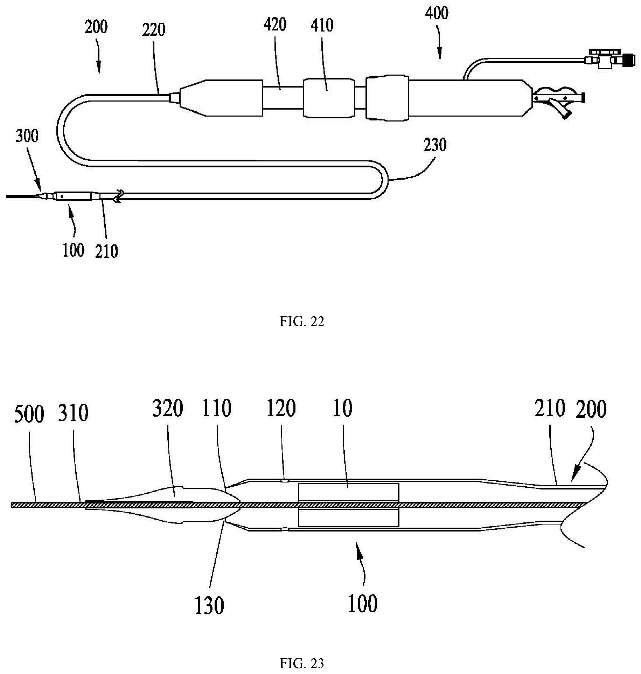

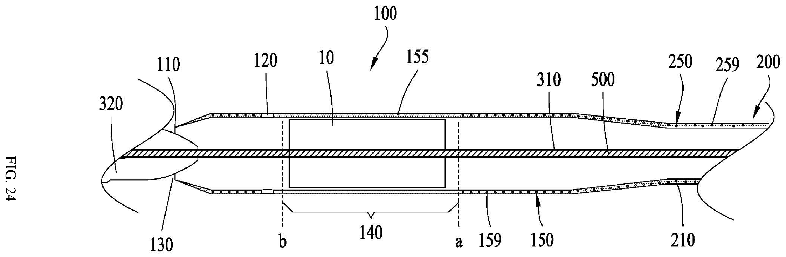

Referring to FIGs. 22 to 24, FIG. 22 is a schematic structural diagram of the delivery device according to an embodiment of the present disclosure. FIG. 23 is a partially enlarged cross-sectional view of a distal end of the delivery device in FIG. 22. and FIG. 24 is a further enlarged view of the container of the delivery device in FIG. 23.

The delivery device includes a container 100, an outer catheter 200, a release mechanism 300, a control mechanism 400 and a guidewire 500. The container 100 and the control mechanism 400 are distributed at two ends of the outer catheter 200. The release mechanism 300 extends through the container 100 and the outer catheter 200 from the inside and is connected to the control mechanism 400. The control mechanism 400 is used for the user to hold and manipulate the release mechanism 300. The guidewire 500 is used to guide the outer catheter 200.

The container 100 is a cylindrical tube structure, and the tube wall encloses to form an accommodating cavity. One end of the container 100 has a tapered outlet 110, and the other end is communicated with the outer catheter 200. In the present embodiment, the outer diameter of the container 100 is greater than the outer diameter of the outer catheter 200, so as to meet the accommodation requirements of the implant 10 as much as possible, while minimizing the overall tube diameter, improving its passing performance, and reducing damage to human tissue during implantation. Of course, in other embodiments, the outer diameter of the container 100 may also be equal to the outer diameter of the outer catheter 200. In the present embodiment, a first metal wire mesh 150 for increasing strength is further provided in the tube wall of the container 100. The first metal wire mesh 150 is formed by weaving a plurality of front segment metal wires 159. The front segment metal wires 159 can be an elastic metal wires, such as a stainless steel wire, a nitinol wire, or a copper wire. The first metal wire mesh 150 is disposed around the side wall of the container 100 as a whole, but does not completely cover the side wall of the container 100 because the first metal wire mesh 150 is not a complete cylindrical structure, but is provided with an opening area 155 at its midsection. The opening area 155 is the entire cylindrical surface area between the position a and the position b. There is no metal wire passing through the opening area 155, thereby forming a signal transmission window 140. The signal transmission window 140 is not a physical opening formed by missing material of the container 100, but an intangible opening that does not hinder wireless signal transmission. The first metal wire mesh 150 itself is used to increase the strength of the container 100, but from an electrical perspective, its mesh structure forms an electromagnetic shielding cover that shields electromagnetic signals. However, there are no metal wires or other shielding structures in the opening area 155, so electromagnetic signal transmission may be not hindered, and therefore, the opening area 155 can be referred to as the signal transmission window 140. In other words, any shielding structure that may hinder signal transmission is not arranged in the signal transmission window.

In the present embodiment, the first metal wire mesh 150 includes two mutually independent mesh cylinders on two sides of the signal transmission window 140, that is, these two mesh cylinders are formed by respectively weaving two groups of metal wires, and at the position a, these metal wires are connected and fixed to each other. The weaving is continued using the same or different number of metal wires from the position b.

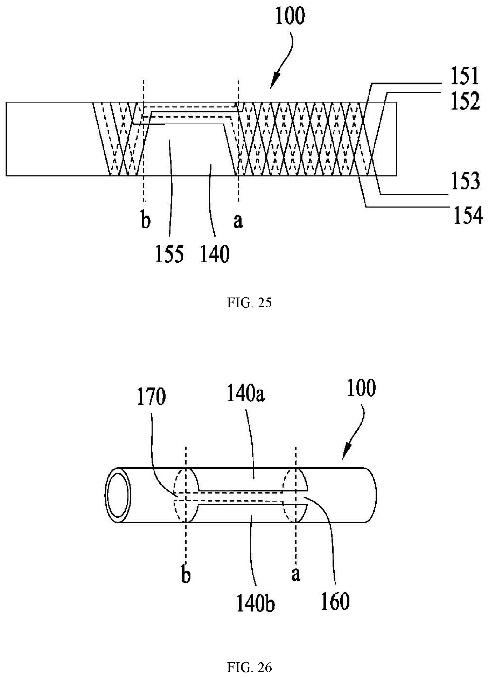

In the alternative embodiment shown in FIG. 25, the first metal wire mesh 150 is formed by weaving the same group of metal wires, which include a first metal wire 151, a second metal wire 152, a third metal wire 153, and a fourth metal wire 154. The first metal wire 151 and the second metal wire 152 are helically wound counterclockwise around the tube body, and the third metal wire 153 and the fourth metal wire 154 are wound clockwise around the tube body. At the position a, the metal wires are no longer helically wound, but continues to extend parallel to the axial direction. After the metal wires extend by a predetermined distance, it continues to be helically wound at the position b. Moreover, when the four metal wires extend in parallel, the circumferential distance is relatively small, so the area occupied is also relatively small. This arrangement does not require cutting the metal wires at the position a to end the weaving, but the same group of metal wires can be used to continue the weaving at the position b, thereby simplifying the weaving process without significantly reducing the strength of the tube segment where the signal transmission window 140 is disposed. Between the position a and the position b, the metal wires extend in parallel, so it does not form a woven structure and has little impact on wireless signal transmission. Moreover, the metal wires are not broken between the position a and the position b, which may not significantly weaken the strength of the container 100 at this location.

In some other alternative embodiments, the metal wires extend in parallel to each other between the position a and the position b and are uniformly distributed in the circumferential direction, forming a non-woven area without the need to be concentrated in a narrower area. As long as the metal wires do not cross and have an appropriate distance, the non-woven area would not significantly affect signal transmission, thus forming a signal transmission window.

In some other alternative embodiments, between the position a and the position b, the metal wires, while extending in the axial direction, are more densely arranged or woven into a mesh belt on one side. At this time, the metal wires may have a certain impact on signal transmission, but can still make way for one or more large opening areas without metal mesh or metal wire distribution, and the opening area forms a signal transmission window.

In other embodiments, the metal wires preceding the position b can also be completely omitted; or alternatively, no metal wire is arranged in the tube wall of the container 100, and then the entire tube wall of the container forms a signal transmission window, which is not limited in the present disclosure.

Preferably, the tube wall of the container 100 is further provided with a side hole 120, and the side hole 120 is located at a distal end of the signal transmission window 140. The side hole 120 can be one or more. In trans-arterial interventional surgery, blood can enter the container 100 via the side hole 120, allowing the sensor in the container 100 to measure physiological parameters such as blood pressure.

The outer catheter 200 is a slender catheter, and includes a catheter body 230, a first end 210 and a second end 220. The outer catheter 200 is a slender hose made of a polymeric material, and the catheter body 230 is provided therein with a second metal wire mesh 250 formed by rear segment metal wires 259. The second metal wire mesh 250 is used to enhance the anti-torsional performance of the outer catheter 200. The rear segment metal wires 259 can be an elastic wire such as a stainless steel wire. The outer catheter 200 is coaxially communicated with the container 100 through the first end 210, and is connected to the control mechanism 400 through the second end 220. Preferably, the front segment metal wires 159 and the rear segment metal wires 259 belong to the same group of metal wires. That is, the metal wires forming the second metal wire mesh 250 extend to the container 100 and are woven to form the first metal wire mesh 150.

The release mechanism 300 includes an inner catheter 310 and a plug 320. The inner catheter 310 is a slender catheter with a diameter smaller than that of the outer catheter 200. The inner catheter 310 passes through the outer catheter 200 and the container 100 from the interior of the outer catheter 200. The plug 320 has a spindle-shaped structure and is provided with a central through-hole along the length direction. The plug 320 is sleeved over the inner catheter 310 via the central through-hole and is located at the outlet 110 of the container 100. One end of the plug 320 can be partially inserted into the outlet 110 to block the outlet 110. A portion of the inner catheter 310 that is located within the container 100 is used for connecting the implant 10. When the inner catheter 310 moves axially relative to the container 100, the inner catheter 310 can drive the implant 10 to move axially relative to the container 100. The plug 320 can also move axially with the inner catheter 310 so as to block the outlet 110 or is partially detached from the outlet 110 to form an annular top clearance 130. The top clearance 130 is used to communicate the interior with the exterior of the container 100. In trans-arterial interventional surgery, blood can enter the container 100 via the top clearance 130, allowing the sensor in the container 100 to measure physiological parameters such as blood pressure. Of course, through the side hole 120 provided on the tube wall of the container 100 as mentioned above, blood can enter the container 100 without moving the inner catheter 310. Therefore, the provision of the side hole 120 can simplify surgical operations and prevent damage to tissues in the body due to the movement of the inner catheter 310 during delivery.

The release mechanism 300 is configured such that the inner catheter 310 can move relative to the container 100 between a first position and a second position in an axial direction. When the inner catheter 310 is at the first position, the implant 10 is located inside the container 100 and corresponds to the signal transmission window. During the process of moving the inner catheter 310 from the first position to the second position, the implant 10 is disengaged axially from the outlet 110 of the container 100 and is placed at the target position. Furthermore, the release mechanism 300 is configured such that when the implant 10 is located within the container 100 and corresponds to the signal transmission window, the side hole 120 is located at a sensing end (e.g., a distal end) of the wireless sensor of the implant 10, thereby allowing blood entering via the side hole 120 to directly contact the implant 10, thereby enabling the sensor in the implant 10 to directly measure physiological parameters.

The control mechanism 400 can be a handle. The control mechanism 400 is used to control the axial relative positional relationship between the outer catheter and the inner catheter in the release mechanism 300. In the present embodiment, the control mechanism 400 includes an actuating member 410 and an auxiliary member 420. The axial position of the auxiliary member 420 is fixed, and actuating member 410 is capable of moving axially on the auxiliary member 420. The actuating member 410 is also connected to the outer catheter 200, so that the actuating member 410 can drive the outer catheter 200 to move axially when the actuating member 410 moves axially. The proximal end of the inner catheter 310 is fixed in the control mechanism 400 and maintains an unchanged axial relative position with the auxiliary member 420. In this way, moving the outer catheter forward or backward in the axial direction can cause axial relative movement between the inner catheter 310 and the outer catheter 200, allowing the implant fixed to the inner catheter 310 to move axially relative to the container at the end of the outer catheter, enabling the implant to be arranged on the inner catheter 310 before implantation, and to be disengaged from the opening of the container in an axial direction when the implant reaches the target. Those skilled in the art can understand that in other embodiments, the control mechanism 400 can also include an actuating member connected to the inner catheter, to achieve axial relative movement between the inner catheter and the outer catheter by actuating the inner catheter and relatively fixing the outer catheter. Since various known catheter handle structures can achieve relative position control between the outer catheter and the inner catheter, the present application will not describe this in detail.

The diameter of the guidewire 500 is smaller than that of the inner catheter 310. The guidewire 500 is a slender metal wire. The inner catheter 310 is sleeved over the guidewire 500. The guidewire 500 has a relatively high support strength and a small diameter, so that the guidewire 500 is easier to bend in the human body vessels and provides guide support.

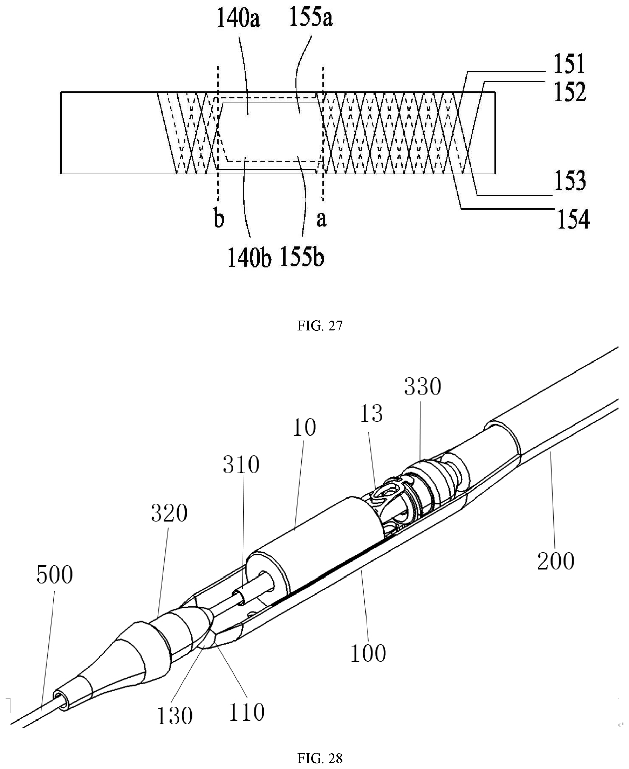

Referring to FIGs. 26 and 27, FIG. 26 is a schematic diagram of distribution of the first metal wire mesh on the surface of the container according to another embodiment of the present disclosure, FIG. 27 is a schematic structural diagram of the first metal wire mesh in FIG. 26. In the present embodiment, the first metal wire mesh includes a first opening area 155a and a second opening area 155b, thereby forming a first signal transmission window 140a and a second signal transmission window 140b. Specifically, the first metal wire 151 and the third metal wire 153 extend parallel to the axial direction from the first side of the container 100 (the second channel 170 in FIG. 26, or the upper side in FIG. 27) at the position a, and the second metal wire 152 and the fourth metal wire 154 extend parallel to the axial direction from the second side of the container 100 (the first channel 160 in FIG. 26, or the lower side in FIG. 27) at the position a. In this way, the first opening area 155a and the second opening area 155b are formed.

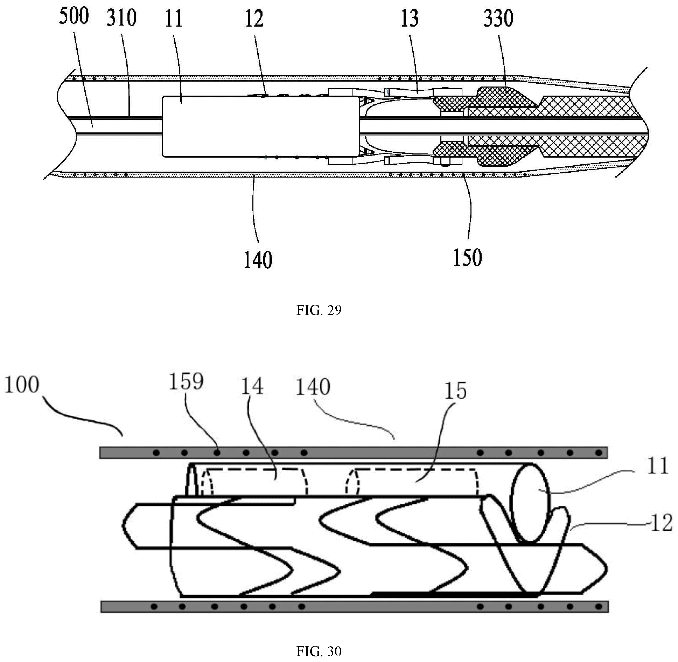

Referring to FIGs. 28 to 30, FIG. 28 is a schematic diagram of the internal structure of the distal end of the medical system according to an embodiment of the present disclosure, FIG. 29 is a partial sectional view of the distal end of the medical system in FIG. 28, and FIG. 30 is a schematic diagram showing the compressed state of the implant in the medical system.

The medical system comprises an implant 10 and a delivery device. The implant 10 has a sensor 11. The sensor 11 is used to measure one or more parameters selected from temperature, flow, pressure, acceleration, vibration, pH value, conductivity, dielectric constant, composition, component content, and acoustic wave. The delivery device is used to accommodate and deliver the implant. Wherein the delivery device can be the delivery device according to any of the embodiments described above.

In a possible embodiment, the implant 10 is a combination of a shunt and a wireless sensor, the wireless sensor is a pressure sensor, and the shunt is compressed within the container and can be released by the release mechanism to the outside of the container. Specifically, the implant 10 includes a wireless sensor 11 and an expandable shunt 12. The wireless sensor 11 is used to measure blood pressure. The sensor 11 includes a pressure detection element 14 and a wireless signal transmission element 15. The wireless signal transmission element is aligned with the signal transmission window 140. The shunt 12 is used for placement in the interatrial septum to guide blood flow from the left atrium to the right atrium. The inner catheter 310 is further provided with an axial limiting member 330. The implant 10 includes a hook member 13 which is connected onto the axial limiting member 330 to maintain a constant relative position with the inner catheter 310 during implantation. After reaching the target position, as the implant 10 is freed from the constraint of the container 100, the hook member 13 may expand together with the shunt 12 to return to its original shape. During this radial expansion, the hook member 13 may disengage from the axial limiting member 330. Specifically, the axial limiting member 330 has a limiting groove, while the hook member 13 has a limiting tab. When the hook member 13 is in a compressed state, the limiting tab is radially and separably engaged with the limiting groove. During the restoration process of the hook member 13, the limiting tab disengages from the limiting groove, thereby also being separated from the axial limiting member 330 in the axial direction.

In a possible embodiment, the hook member 13 can also be a rod-shaped element that does not expand itself, but can follow the compression and expansion of the shunt 12 to approach or move away from the axial limiting member 330, thereby achieving engagement or separation. It can be understood that in order to clearly display individual elements, the distance between the shunt 12 and/or the hook member 13 and the inner wall of the container is enlarged in FIG. 29.

The implant 10 can be pre-positioned in the container 100 after being compressed, or can be temporarily loaded into the container 100 during use. When the shunt 12 is compressed, it is radially compressed into a concave structure with a C-shaped cross-section, and this concave structure can partially surround the sensor 11.

During a cardiovascular interventional surgery, physiological parameters and indicator curve changes in various parts of the cardiac cavity and blood vessels are measured accurately and continuously throughout the implantation process, which helps the physician to judge the patient's cardiac function, cardiac load, and pulmonary hemodynamic load, etc., and meanwhile provides reference for guiding surgical strategies and evaluates postoperative efficacy in real time.

In an implantation surgery such as atrial shunt, a shunt with a wireless pressure sensor can be implanted into the interatrial septum by the delivery device described above. The delivery device described above not only ensures the strength of the interventional catheter, but also allows the pressure sensor to transmit wireless signals to the outside of the human body in real time, achieving the effect of real-time and accurate monitoring.

The interventional surgery using the delivery device or the medical system described above also has the following advantages in clinical practice:
1. In emergency situations during surgery, hemodynamic indicators of various parts of the cardiac cavity and blood vessels can be directly measured without the need for additional catheterization, reducing surgical risks and minimizing patient pain.
2. When the wireless sensor is a pressure sensor, there is no need to additionally place a right heart catheter to measure pressure changes in the superior and inferior veins, right atrium, pulmonary artery, and pulmonary arterioles, thereby reducing patient trauma. It may also be possible to reduce the complexity of surgery and the impact of intracardiac catheterization on intraoperative operations, and also reduce surgical time and costs.
3. When the wireless sensor is a pressure sensor, the average pressure values and change curves of different cardiac cavities can be used to assist in determining the position of the implant delivery device in the cardiac cavity. This can assist the physician in determining the position of the delivery system within the cardiac cavity (right atrium, left atrium, superior and inferior vena cava) without image guidance, thereby simplifying the specific operations of instrument release during surgery and reducing ray radiation to the patient.
4. When the wireless sensor is a pressure sensor, compared with the data (such as left atrial pressure) measured by traditional right heart catheterization, the data of the cardiac cavity pressure obtained through the delivery device of the present disclosure is more accurate and real-time. This avoids interference from factors such as deviations and time differences caused by pressure transmission. The cardiac cavity pressure can be measured immediately after surgery to directly evaluate the postoperative treatment effect and provide the physician with the basis for treatment strategies.

The above descriptions are merely illustrative specific embodiments of the present disclosure and are not intended to limit the scope of the present disclosure. Any equivalent changes and modifications made by those skilled in the art without departing from the concepts and principles of the present disclosure shall fall within the scope of the present disclosure.

## Claims

1. A delivery device for delivering an implant to a target tissue via a lumen or vessel in a human body, wherein the delivery device comprises:
a container comprising an accommodating cavity for accommodating the implant and an outlet for discharging the implant;
an outer catheter connected to and communicated with the container;
a release mechanism comprising an inner catheter and a control mechanism, wherein the inner catheter extends through the container and the outer catheter, and is configured to connect with the implant, wherein the control mechanism is configured to control an axial relative positional relationship between the inner catheter and the outer catheter.

2. The delivery device according to claim 1, wherein the delivery device comprises an end structure provided at a distal end of the inner catheter and configured to selectively block or expose the outlet; wherein the outer catheter and the container are coaxially disposed, and the container is axially movable relative to the end structure to cause the end structure to block or expose the outlet; when a distance between the container and the end structure is smaller than a preset value, a distance between the end structure and the implant remains unchanged, and when the distance between the container and the end structure is greater than or equal to the preset value, the implant is released to the target tissue; wherein the implant comprises a stent made of a shape memory material, the stent is adapted to be sleeved over the inner catheter, and the stent is capable of self-expanding to a set width after being separated from the inner catheter.

3. The delivery device according to claim 2, wherein the end structure comprises a balloon which has a first operating state of being in an inflated configuration and a second operating state of being in a deflated configuration, and when in the first operating state, the balloon is movable axially relative to the container to block or expose the outlet; during the process of separating the implant from the inner catheter, the balloon remains closer to a distal end of the implant than a proximal end of the implant and maintains an unchanged relative axial position with respect to the implant; wherein after the implant is separated from the inner catheter, the balloon is capable of directly expanding the stent on a withdrawl path.

4. The delivery device according to claim 3, wherein a length of the balloon is greater than or equal to a length of the stent; and/or, a nominal width of the balloon after inflation is greater than or equal to the set width of the stent.

5. The delivery device according to claims 2 or 3, wherein the delivery device further comprises a radiopaque marker disposed on the end structure; and/or, the end structure is made of a material with a radiopaque function.

6. The delivery device according to claims 2 or 3, wherein along a direction from the distal end of the inner catheter to a proximal end of the inner catheter, both ends of the end structure respectively form a pre-dilation guide portion and a post-dilation guide portion; along a direction from the post-dilation guide portion to the pre-dilation guide portion, a cross-section of the pre-dilation guide portion gradually decreases; and/or, along a direction from the pre-dilation guide portion to the post-dilation guide portion, a cross-section of the post-dilation guide portion gradually decreases.

7. The delivery device according to claims 2 or 3, wherein along a direction from the distal end to a proximal end of the inner catheter, the inner catheter comprises a first extension segment, a second extension segment and a third extension segment, an extension direction of the second extension segment is different from that of the first extension segment and the second extension segment, wherein an axis of the first extension segment is offset to one side of an axis of the container; wherein a main body of the implant is disposed on the first extension segment, and the stent is sleeved over the first extension segment.

8. The delivery device according to claim 7, wherein a first port portion and a second port portion are formed at both ends of the end structure along a direction from the distal end of the inner catheter to the proximal end of the inner catheter; the first port portion and the second port portion are disposed coaxially with the axis of the container; or the first port portion is disposed coaxially with the axis of the container, and the second port portion is disposed coaxially with the axis of the first extension segment; or the first port portion and the second port portion are disposed coaxially with the axis of the first extension segment; or the first port portion is disposed coaxially with the axis of the first extension segment, and the second port portion is disposed coaxially with the axis of the container.

9. The delivery device according to claim 8, wherein the end structure comprises a balloon, the first port portion and the second port portion of the balloon are respectively sleeved over the inner catheter, and the main body of the balloon is disposed coaxially with the container;
wherein the length of the balloon is greater than or equal to the length of the stent; and/or, a nominal width of the balloon after inflation is greater than or equal to the set width of the stent, so that after the implant is separated from the inner catheter, the balloon is capable of directly expanding the stent on a withdrawl path.

10. The delivery device according to claim 7, wherein the delivery device further comprises a first connection structure provided on the inner catheter, and the implant is provided thereon with a second connection structure;
when the implant is within the container, the implant is connected to the first connection structure via the second connection structure to maintain an axial relative position between the implant and the inner catheter;
when the implant disengages from the container, the second connection structure and the first connection structure are capable of automatically separating from each other to release the implant.

11. The delivery device according to claim 10, wherein a connecting member is provided on the inner catheter, wherein the connecting member is accommodatable within the container and the first connection structure is provided on the connecting member; wherein the connecting member comprises a connecting seat on which an eccentric channel for the inner catheter to pass through is formed; wherein the connecting seat is provided to extend along a first preset direction, wherein a first included angle β is formed between the first preset direction and an axis of the eccentric channel, wherein the first included angle β ranges from 0° to 60°.

12. The delivery device according to claim 11, wherein the outer catheter has a first bending portion, and the inner catheter has a second bending portion, and when the end structure blocks the outlet, the first bending portion and the second bending portion are correspondingly disposed;
wherein along the direction from the distal end of the inner catheter to the proximal end of the inner catheter, the connecting seat comprises a first seat body and a second seat body connected with each other, wherein the first seat body is cylindrical, and the first connection structure is disposed on the first seat body;
wherein the second seat body is frustum-shaped, the eccentric channel extends through the first seat body and the second seat body, the second seat body has a first end and a second end, and a radial dimension of the first end is greater than that of the second end, and the first end of the second seat body is connected to the first seat body.

13. The delivery device according to claim 12, wherein the second end of the second seat body is formed with an orifice end face which is perpendicular to the axis of the eccentric channel, and the eccentric channel extends through the orifice end face; wherein the second seat body is further provided with an avoidance end face, wherein at least a part of the avoidance end face is connected to the orifice end face, and the remaining part of the avoidance end face is disposed to be away from the proximal end of the inner catheter, wherein a second included angle α is formed between the avoidance end face and the vertical direction, wherein the second included angle α ranges from 0° to 60°.

14. The delivery device according to claim 11, wherein the inner catheter further comprises a fourth extension segment, wherein the fourth extension segment is located between the second extension segment and the third extension segment, the fourth extension segment is configured to extend through the eccentric channel, wherein an included angle between the fourth extension segment and the axis of the container is smaller than that between the second extension segment and the axis of the container.

15. The delivery device according to claim 14, wherein an end channel is formed on the end structure, the inner catheter further comprises a fifth extension segment, wherein the fifth extension segment is disposed on the other side of the first extension segment relative to the second extension segment, and the fifth extension segment extends through the end channel; wherein the inner catheter has an inner cavity for placement of a guidewire.

16. The delivery device according to claim 11, wherein a flow guide structure is provided on the connecting member, wherein the flow guide structure communicates accommodating cavities of the container that are located on both sides of the connecting member; wherein the first preset direction is parallel to the axial direction of the container or forms a third included angle γ with the axial direction of the container, wherein the third included angle γ ranges from 0° to 60°.

17. The delivery device according to claim 2, wherein the end structure comprises a balloon, the inner catheter is further provided thereon with a flow-through structure, wherein the flow-through structure has an injection channel that is communicated with an inner cavity of the balloon.

18. The delivery device according to claim 1, wherein the container is provided with a signal transmission window;
wherein the outer catheter has a catheter body, a first end and a second end, wherein the outer catheter is connected to and coaxially communicated with the container through the first end; and
wherein the release mechanism extends through the outer catheter and the container, and the release mechanism is configured to connect with the implant and control an axial relative positional relationship between the implant and the container;
wherein the implant comprises a wireless sensor, and the release mechanism is configured to keep the implant in the container and align the wireless sensor with the signal transmission window.

19. The delivery device according to claim 18, wherein no shielding structure that hinders signal transmission is provided in the signal transmission window, and an outer diameter of the container is greater than that of the outer catheter.

20. The delivery device according to claim 18, wherein the container comprises front segment metal wires, wherein the front segment metal wires are provided in a side wall of the container and form a first metal wire mesh;
wherein the first metal wire mesh is provided with an opening area, wherein no front segment metal wire passes through the opening area, and the opening area forms the signal transmission window; or the first metal wire mesh is provided with a non-woven area, wherein the front segment metal wires do not intersect in the non-woven area, and the non-woven area forms the signal transmission window.

21. The delivery device according to claim 20, wherein when the first metal wire mesh is provided with the opening area, the opening area is formed by the front segment metal wires giving way.

22. The delivery device according to claim 21, wherein the front segment metal wires change their extending directions and extend axially for a predetermined distance without intersecting each other, so as to give way to form the opening region.

23. The delivery device according to claim 22, wherein the outer catheter comprises rear segment metal wires, wherein the rear segment metal wires are provided in a side wall of the outer catheter and form a second metal wire mesh, wherein the second metal wire mesh extends to the container to form the first metal wire mesh.

24. The delivery device according to any of claims 18 to 23, wherein the number of the signal transmission windows is one or more.

25. The delivery device according to claim 18, wherein the container is provided with a side hole, wherein the side hole is located at a distal end of the signal transmission window, and when the wireless sensor is aligned with the signal transmission window, the side hole corresponds to a sensing end of the wireless sensor.

26. The delivery device according to claim 18, wherein a plug is provided at an outlet of the container, the plug is connected to the release mechanism, and the plug is drivable by the release mechanism to be displaced axially to form a top gap with the outlet.

27. The delivery device according to claim 18, wherein the release mechanism comprises an inner catheter and an axial limiting member, wherein the inner catheter extends through the container and the outer catheter, and is configured to connect with the implant, and the axial limiting member is configured to maintain an axial relative positional relationship between the implant and the inner catheter.

28. The delivery device according to claim 27, wherein the release mechanism is configured such that the inner catheter is movable relative to the container between a first position and a second position in an axial direction, and when the inner catheter is in the first position, the implant is located inside the container and the wireless sensor corresponds to the signal transmission window, during a process of the inner catheter moving from the first position to the second position, the implant is disengaged axially from an opening of the container.

29. The delivery device according to claim 18, wherein the wireless sensor is configured to measure one or more parameters selected from temperature, flow, pressure, acceleration, vibration, pH value, conductivity, dielectric constant, composition, component content, and acoustic wave.

30. A medical system comprising an implant and a delivery device according to any one of claims 1 to 17, wherein the delivery device is configured to accommodate and deliver the implant.

31. The medical system according to claim 30, wherein the medical system further comprises a guidewire for being disposed within the inner catheter, wherein the implant comprises a wireless sensor and a stent, wherein the wireless sensor and the stent are provided side by side.

32. A medical system comprising:
an implant comprising a wireless sensor; and
a delivery device configured to accommodate and deliver the implant;
wherein the delivery device is the delivery device according to any of claims 18 to 29.

33. The medical system according to claim 32, wherein the implant comprises an expandable shunt, the wireless sensor is a pressure sensor, wherein the pressure sensor is connected with the shunt, and the shunt is compressed within the container and releasable by the release mechanism to the outside of the container.

34. The medical system according to claim 33, wherein the pressure sensor is provided side by side with the shunt and outside of the shunt, and the pressure sensor is compressed by the container along a radial direction of the shunt to be partially surrounded by the shunt.

35. The medical system according to claim 33, wherein when the release mechanism comprises an inner catheter and an axial limiting member, the implant further comprises a hook member, wherein the hook member is connected to the axial limiting member when the implant is compressed, and disengages from the axial limiting member when the implant expands.

36. The medical system according to claim 33, further comprising a signal receiving device for receiving a signal from the wireless sensor in the implant.
